# EUROPEAN PATENT APPLICATION

(11) **EP 1 134 280 A1**
(43) Date of publication of application: **19.09.2001**
(21) Application number: 00200866.2
(22) Date of filing: 10.03.2000
(51) Int. Cl.: C11D 17/00, C11D 17/02, C11D 17/04, C11D 3/50, A61L 9/05

(54) **Deodorization block**

(71) Applicant: Grahmbeek, Tamar Vanessa, 1181 GE Amstelveen (NL); Grahmbeek, Marit Astrid, 1935 BN Egmond Binnen (NL)
(72) Inventor: Grahmbeek, Tamar Vanessa, 1181 GE Amstelveen (NL); Grahmbeek, Marit Astrid, 1935 BN Egmond Binnen (NL)
(74) Representative: Van Assen, Jan Willem Bernard

(57) **Abstract**

The deodorization block is applied in toilets.

The deodorization block comprises a relatively small amount gas developing substance and perfume and is provided with an easily to open moisture resistant envelope. After elimination of the easily to open envelope the deodorization block is deposited in the toilet bowl, preferably prior to each use of the toilet.

## Description

The invention relates to a deodorization block comprising one or more compounds which release with water gases and perfumes for masking of odours.

The invention relates as well to methods for manufacturing of the deodorization block as well to application thereof.

From AU-649491 (filed October 31st, 1990, published May 7th, 1992, publication date examined (accepted) patent application May 26th, 1994) a deodorization block is known useful for controlling and neutralizing of bad odours in toilets and so on.

The said deodorization block contains besides sodium percarbonate, from which on contact with water hydrogen peroxide (H₂O₂) is released and perfume, ethoxylated fatty alcohol detergent and calcium stearate.

Application of the combination of calcium stearate and ethoxylated fatty alcohol detergent provides the deodorization block with a slow and solubilizing action.

The last fact is mentioned in the before last paragraph of page 6 (the fore last page of the claims page) of AU-649491.

In certain cases now, slow release of odour combatting substances with use of toilets can be less desirable.

Therefore, according to the invention, addition of means which slow down the release of odour combatting ingredients is omitted.

The invention is based on the thought, that the toilet bowl is about 100 times smaller than the whole toilet space and that depositing of an odour combatting tablet in the toilet bowl, preferably each time before use of the toilet is very effective.

The odour or stench namely is being combatted before this has dispersed through the whole toilet space.

Because users convenience of the deodorization block is important, this is provided with an easily to open envelope.

With easily to open is meant for instance, an envelope of a foil compatible with the odour combatting means, provided with an opening pull cord, pull band, or pull lip.

According to a preferable embodiment the deodorization block is provided with means or reducing the density below ≤1, in order that the development of gas and perfume occurs as much as possible in the atmosphere of the toilet bowl.

It is namely undesirable that a great part of the perfume dissolves in de wash-out water of the toilet.

The usable density reducing means can for instance be composed of a floating body.

The floating body can be provided with indications as top- and bottom side, with respect to eventually preferable exposition of the odour combatting substances to water.

Preferably the density reducing means are made of biodegradable material.

The deodorization block can comprise as with water gas developing compounds one or more compounds selected from the groups a, b and c mentioned below.
a) hydrogen peroxide developing compounds, percarbonates or perborates for instance;
b) combinations of compounds which in water develop chlorine dioxide, or
c) combinations of compounds which in water develop carbon dioxide.

With respect to the foregoing with respect to a), b) and c) respectively, there is referred to:
a) the before mentioned AU-649491,
b) DE-A-2712574 and JP-A-91/285801,
c) DE-A-2712574.

Suitable perfumes for combatting odour are the substances mentioned below in US-A-5559271, there used for masking the odour of mercaptanes:
one or more substances selected from
wintergreen (oil), vanillin, ethyl vanillin, o-vanillin, vanillinic acid, oleoresin vanillin, menthol, piperitone, menthyl acetate, neomenthol, pulegone, spearmint ((-)-carvone), carvyl acetates, cornmint, scotch mint, peppermint, α-pinene, β-pinene, linalool, nerol, α-terpinene, menthofuran, β-terpinene, Y-terpinene, myrcene, geraniol, geranial, neral, citronellal, menthone, isomenthone, 1,8-cineole, ascaridole, borneol flavone, terpinolene, sabinene, camphene, citronellol, cinnamic aldehyde, cinnamic acids, methyl cinnamate, benzyl cinnamate, cinnamyl acetate, cinnamyl cinnamate (styracin).

According to a further advantageous embodiment the deodorization block contains a desintegration means, which causes that on contact with water it desintegrates.

An example of a desintegration means is sodium carboxy methyl cellulose, which on contact with water swells, which causes desintegration.

Because the compositions of the deodorization block in a moist atmosphere are often not stable, it is recommended to incorporate in the composition a drying agent.

A suitable drying agent is anhydrous potassium carbonate.

As earlier mentioned, the invention relates to the manufacture thereof. To this end the odour combatting means and eventually gas developing compounds and further additives are subsequently mixed, at which carboxy methyl cellulose is added, subsequently formed into tablets or otherwise divided in relatively small portions, possibly provided with a floating body and subsequently packed in a moisture resistant package.

Preferably the packages of the relatively small portions have to be easily to open.

Finally the invention relates to the application of the package relatively small portions of the deodorization block.

After opening of a separate deodorization block this is deposited in a toilet bowl preferably before toilet use.

## Claims

1. Deodorization block, comprising one or more compounds which with water release gases and perfume for masking of odours, **characterized in that**, the deodorization block contains no means for slowing down the release of the active ingredients and is provided with an easily to open envelope.

2. Deodorization block according to claim 1, **characterized in that**, this comprises means which reduce the density thereof to ≤1.

3. Deodorization block according to claim 2, **characterized in that**, this is provided with a floating body.

4. Deodorization block according to claim 3, **characterized in that**, this is provided with indications of bottom- and top side respectively.

5. Deodorization block according to any of claims 2-4, **characterized in that**, the means consist of biodegradable material.

6. Deodorization block according to any of the foregoing claims, **characterized in that**, this contains one or more of
a) hydrogen peroxide developing compounds (percarbonates or perborates for instance);
b) combinations of compounds which in water develop chorine dioxide;
c) combinations of compounds which in water develop carbon dioxide.

7. Deodorization block according to any of the foregoing claims, **characterized in that**, this comprises one or more perfumes selected from
wintergreen (oil), vanillin, ethyl vanillin, o-vanillin, vanillinic acid, oleoresin vanillin, menthol, piperitone, menthyl acetate, neomenthol, pulegone, spearmint ((-)-carvone), carvyl acetates, cornmint, scotch mint, peppermint, α-pinene, β-pinene, linalool, neral, α-terpinene, menthofuran, β-terpinene, Y-terpinene, myrcene, geraniol, geranial, neral, citronellal, menthone, isomenthone, 1,8-cineole, ascaridole, borneol flavone, terpinolene, sabinene, camphene, citronellol, cinnamic aldehyde, cinnamic acids, methyl cinnamate, benzyl cinnamate, cinnamyl acetate, cinnamyl cinnamate (styracin).

8. Deodorization block according to any of the foregoing claims, **characterized in that**, this comprises a desintegrating agent, such as for instance sodium carboxy methyl cellulose.

9. Deodorization block according to any of the foregoing claims, **characterized in that**, this is provided with a drying agent, such as for instance anhydrous potassium carbonate.

10. Method for the manufacture of the deodorization block according to any of the claims 1-9, **characterized in that**, the other combatting agents are mixed, eventually provided with sodium carboxy methyl cellulose, subsequently tableted and thereby eventually provided with a floating body and packed in a moisture resistant envelope.

11. Application of the deodorization block according to any of the foregoing claims.

12. Package provided with one or more of the deodorization blocks according to claims 1-9.
